# EUROPEAN PATENT APPLICATION

(11) **EP 2 586 438 A1**
(43) Date of publication of application: **01.05.2013**
(21) Application number: 11187170.3
(22) Date of filing: 28.10.2011
(51) Int. Cl.: A61K 31/205, A61K 31/221, A61K 31/70, A61K 31/7072

(54) **Compound useful for preventing cognitive deficit disorders in a new born from HIV-seropositive pregnant female who is in treatment with azidothymidine**

(71) Applicant: SIGMA-TAU Industrie Farmaceutiche Riunite S.p.A., 00144 Roma (IT)
(72) Inventor: Casolini, Paola, 00018 Rome (IT); Koverech, Aleardo, 00165 Rome (IT); Nicolai, Raffaella, 00148 Roma (IT)
(74) Representative: Tagliafico, Giulia

(57) **Abstract**

The present invention relates to acetyl L-carnitine for use in preventing cognitive deficit disorders in a new born from HIV-seropositive pregnant female who is in treatment with azidothymidine.

## Description

### FIELD OF THE INVENTION

The present invention relates to acetyl L-carnitine or one of its pharmaceutically acceptable salts for use in preventing cognitive deficit disorders in a new born from HIV-seropositive pregnant female who is in treatment with azidothymidine (AZT).

### BACKGROUND OF THE INVENTION

Human immunodeficiency virus (HIV) is a latent virus that causes acquired immunodeficiency syndrome (AIDS), a condition in humans in which progressive failure of the immune system allows life-threatening opportunistic infections and cancers to thrive.

HIV infection in humans is considered pandemic by the World Health Organization (WHO). From its discovery in 1981 to 2006, AIDS killed more than 25 million people. A disproportionate number of AIDS deaths occur in Sub-Saharan Africa, retarding economic growth and exacerbating the burden of poverty. Treatment with antiretroviral drugs reduces both the mortality and the morbidity of HIV infection. The four major routes of transmission are unsafe sex, contaminated needles, breast milk, and transmission from an infected mother to her baby at birth (perinatal transmission).

The transmission of the virus from mother to child can occur in utero (during pregnancy), intrapartum (at childbirth), or via breast feeding. In the absence of treatment, the transmission rate up to birth between the mother and child is around 25%. (N. Engl. J. Med.2004, 351 (3): 289-292).

However, where combination antiretroviral drug treatment and Cesarian section are available, this risk can be reduced to as low as one percent. Postnatal mother-to-child transmission may be largely prevented by complete avoidance of breast feeding; however, this has significant associated morbidity.

Azidothymidine (AZT) (also called ZDV) is a nucleoside analog reverse-transcriptase inhibitor (NRTI), a type of antiretroviral drug used for the treatment of HIV and/or AIDS.

The use of AZT in the treatment of HIV is already known in the art.

In US 2010273733 a method is disclosed for enhancing the anti-HIV effect of the antiretroviral drug AZT by administering with the AZT, a pharmaceutical composition comprising an aminoalkanol and a glucocorticoid.

AZT was the first approved treatment for HIV. AZT use was a major breakthrough in AIDS therapy in the 1990s that significantly altered the course of the illness and helped destroy the notion that HIV/AIDS was a death sentence. AZT slows HIV spread significantly, but does not stop it entirely.

Current treatment regimens involve lower dosages (e.g., 300 mg) of AZT taken twice a day, almost always as part of highly active antiretroviral therapy (HAART) protocols. AZT is combined with other drugs in order to prevent mutation of HIV into an AZT-resistant form (Biochem Pharmacol 1994, 47 (2): 155-69). AZT is also recommended as part of a regimen to prevent mother-to-child transmission of HIV during pregnancy, labor, and delivery. AZT has been shown to reduce this risk to approximately 8% when given in a three-part regimen during pregnancy, delivery and to the infant for 6 weeks after birth (N Engl J Med 1994, 331 (18): 1173-80).

However, it has been demonstrated that the administration of AZT during pregnancy can lead to toxic side effects on the fetus which develops in a cognitive disorder in the newborn. The most frequent cognitive disorder that can occur in fetuses or young children is mental retardation. It is a condition in which a person has low cognitive abilities due to developmental delays. He or she cannot learn new concepts or function at the same level as other people his or her age. Although the severity of mental retardation can vary, many individuals suffering from said condition do not have the mental skills to safely care for themselves.

Therefore the discovery of agents useful for reducing the toxic side effects in newborn without significantly reducing the pharmacological activity of AZT remains a perceived need in the medical field.

The use of acetyl L-carnitine is already known in the art.

EP 0839033 relates to the use of acetyl L-carnitine in combination with nucleoside-like inhibitors of reverse transcriptase, non-nucleoside inhibitors of reverse transcriptase and inhibitors of HINT protease, for reducing ceramide levels and enhance the activity of the aforesaid antiretroviral drugs in HIV-infected patients.

US 6,037,372 relates to the use of acetyl L-carnitine for the therapeutic treatment or prophylaxis of glutamate-mediated disturbances or diseases selected from cancer, infection with HIV, immunodeficiencies, drug dependencies, headaches, chronic fatigue syndrome, schizophrenic disorders, epilepsy, amyotrophic lateral sclerosis and other motor neuron diseases and peripheral neuropathies, senile and presenile dementias, apoplexy and sequences thereof, cerebrovascular ischaemic diseases, decreased cerebral flow and altered cerebral metabolism, neurodegenerative diseases, Huntington's disease, Parkinson's disease, prion protein diseases, meningoencephalitis, and Chinese restaurant syndrome.

US 5192805 relates to the use of acetyl L-carnitine in the therapeutic treatment of coma.

EP 0808626 relates to the use of acetyl L-carnitine to produce a medicament for the treatment of Alzheimer's disease (AD).

EP 0207011 relates to the use of some alkanoyl L-carnitines for the manufacture of a medicament for therapeutical treatment of idiopathic and induced Parkinsonism, acetyl L-carnitine is mentioned.

Acetyl L-carnitine is a known compound, for which the preparation process is described in US 4,254,053.

None of the publications known in the art mention nor suggest the use of acetyl L-carnitine for preventing cognitive deficit disorders in a new born from HIV-seropositive pregnant female who is in treatment with azidothymidine.

### DESCRIPTION OF THE INVENTION:

It has now been found that acetyl L-carnitine or one of its pharmaceutically acceptable salts is useful in preventing cognitive deficit disorders in a new born from HIV-seropositive pregnant female, which are in treatment with azidothymidine.

What is meant by pharmaceutically acceptable salt of acetyl L-carnitine is any salt prepared by addition of an acid to acetyl L-carnitine inner salt, and which does not give rise to unwanted toxic or side effects. The formation of salts by addition of an acid is well known in pharmaceutical technology.

Non-limiting examples of such salts are chloride, bromide, orotate, aspartate, acid aspartate, citrate, acid citrate, magnesium citrate, phosphate, acid phosphate, fumarate, acid fumarate, magnesium fumarate, glycerophosphate, lactate, maleate and acid maleate, mucate, oxalate, acid oxalate, pamoate, acid pamoate, sulphate, acid sulphate, glucose phosphate, tartrate, acid tartrate, magnesium tartrate, 2-amino ethanesulphonate, magnesium 2-amino ethanesulphonate, methane-sulphonate, choline tartrate, trichloroacetate, and trifluoroacetate.

It is therefore an object of the present invention acetyl L-carnitine for use for preventing cognitive deficit disorders in a new born from HIV-seropositive pregnant female who is in treatment with azidothymidine.

Acetyl L-carnitine is preferably administered daily, at the early stage of pregnancy; in an independent, concomitant or sequential manner respect to the administration of AZT.

Any accepted mode of administration can be used and determined by those skilled in the art. For example, administration may be by various parenteral routes such as subcutaneous, intravenous, intradermal, intramuscular, intraperitoneal, intranasal, transdermal, oral, or buccal routes. Parenteral administration can be by bolus injection or by gradual perfusion over time. Preparations for parenteral administration include sterile aqueous or non-aqueous solutions, suspensions, and emulsions, which may contain auxiliary agents or excipients known in the art, and can be prepared according to routine methods.

The acetyl L-carnitine according to the present invention is in the form of tablets, capsules, suppositories, suspensions or solutions.

It is understood that the dosage administered will be dependent upon the age, sex, health, and weight of the recipient, kind of concurrent treatment, if any, frequency of treatment, and the nature of the effect desired. The dosage will be tailored to the individual subject, as is understood and determinable by one of skill in the art. The total dose required for each treatment may be administered by multiple doses or in a single dose.

The active ingredients of the present invention are familiar to operators in the medical field and already in use.

Their procurement therefore is very easy, inasmuch as these are products which have been on the market now for a long time and are of suitable purity for human administration.

For any compound, the therapeutically effective dose can be estimated initially either in cell culture assays, for example, of neoplastic cells, or in animal models, usually mice or rats.

The animal model may also be used to determine the appropriate concentration range and route of administration. Such information can then be used to determine useful doses and routes for administration in humans.

According to the present invention the dose of acetyl L-carnitine to be administered is from 500 mg to 4 grams/day, a preferred dose is 1-3 grams/day, the most preferred dose is 2 grams/day. The precise effective dose for a human subject will depend upon the severity of the disease state, general health of the subject, age, weight, and gender of the subject, diet, time and frequency of administration, drug combination(s), reaction sensitivities, and tolerance/response to therapy. This amount can be determined by routine experimentation and is within the judgement of the clinician.

### DESCRIPTION OF THE FIGURES

Figure 1: Experimental procedures
Figure 2: Effect of the treatment with AZT, ALC and AZT+ALC on the Expression of hippocampal metabotropic glutamate receptors (mGluR1a, mGluR2/3, mGluR5) in two months old mice (PND60).
Figure 3: Effect of the treatment with AZT, ALC and AZT+ALC on the Expression of hippocampal ionotropic glutamate AMPA receptors (Glu1, Glu2) in two months old mice (PND60).
Figure 4: Effect of the treatment with AZT, ALC and AZT+ALC on the Expression of hippocampal ionotropic glutamate NMDA receptors (NR1) in two months old mice (PND60).
Figure 5: Effect of the treatment with AZT, ALC and AZT+ALC on spatial learning in the Morris Water Maze in two months old mice (PND60).
Figure 6: Effect of the treatment with AZT, ALC and AZT+ALC on delta between restraint stress values and basal values of plasma corticosterone in two months old mice (PND60).
Figure 7: Effect of the treatment with AZT, ALC and AZT+ALC on brain oxidative stress in newborn (PND0) (7A) and in two months old mice (PND60) (7B).

The following non limiting examples further illustrate the invention.

### Example 1

### Materials and Methods

### Animals and housing conditions

Adult male (n=28) and adult virgin female (n=56) mice of outbred Swiss-derived strain (CD-1) were purchased from Charles River (Calco, Italy). Upon arrival at the laboratory, the animals were housed in an air-conditioned room (temperature 20-22°C, relative humidity 55± 5%) with a reversed 12:12 light cycle (light on at 19.00 h, light off at 07.00 h). Water and food were available ad libitum. Females were group-housed (4 per cage) for 7 days to coordinate their estrous cycle. After that, pairs of female mice were housed with a single sexually experienced male mice. Females were daily inspected for the presence of a vaginal plug (pregnancy day 0; GDO) and therefore individually housed in Plexiglass cages (33 x 13 x 14 cm). Pregnancy rate was about 75%. The litters were culled at birth to four females and four males, to maintain adequate litter composition. Only male litter mates were used in this study.

### Experimental procedures

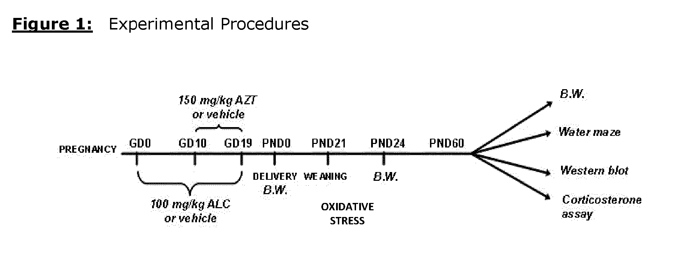

The experimental procedures are designed in Fig 1.

On gestational day 10 (GD10), pregnant mice were randomly assigned, on the basis of the treatment received, to four groups: Saline (Control), 3'-azido-3'-deoxythimidine (AZT), acetyl L-carnitine (ALC) and AZT+ALC. AZT was purchased from Sigma-Aldrich (Milano, Italy) and ALC was synthesized and provided by Sigma Tau Laboratories (Pomezia, Rome, Italy) AZT was dissolved in bidistilled water and ALC in saline.

The treatment consisted in the daily administration of ALC (100 mg/kg) or vehicle (saline) by subcutaneous injection (s.c.; 1.25ml/kg), from GD0 to delivery, and in the twice daily (10:00 h and 17:00 h) administration of AZT (150 mg/kg) or vehicle (water; 3.3 ml/kg) by transoral gavage, from GD10 to delivery.

The Control group was s.c. injected with saline and orally treated twice daily with water. The AZT group was s.c. injected with saline and orally treated twice daily with AZT. The ALC group was s.c. injected with ALC and orally treated twice daily with water. The AZT+ALC group was s.c injected with ALC and orally treated twice daily with AZT.

The day of birth was defined as postnatal day 0 (PND0). Weaning was performed at 21 days of age (PND21) and the male litter mates of each group were housed in two per cage. They were maintained under controlled environmental conditions until 2 months of age (PND60) when they were assigned to glutamate western blot receptor analysis or plasma corticosterone assay or water maze behavioural test.

In order to ascertain the influence of the treatment on the body weight, litter mates of each group were weighed at birth (PND0), after weaning (PND24) and at adult age (PND60).

The experiments were conducted in accordance with the guidelines of the Italian Ministry of Health (D.L. 116/92), the declaration of Helsinki, and the "Guide for the Care and Use of laboratory Animals" as adopted and promulgate by the National Institutes of Health (USA).

### Western blot analysis

A set of mice were sacrificed on PND60 to assess expression of ionotropic and metabotropic glutamate receptors. For the ionotropic receptors we assessed AMPA Glu1 and Glu2 subunit, and NMDA NR1 subunit. For the metabotropic receptors we measured both group I mGluR1 and mGluR5 subtypes and group II mGlu2/3 subtypes. Mice were killed by decapitation and brains rapidly removed; hippocampi were dissected and stored at -80°C. On the day of the experiment, tissue was homogenized at 4°C with a polytron in 500 µl of 100 mM Tris buffer, containing phenylmethylsulfonyl fluoride 1 mM, leupeptin 10 µg/ml and aprotinin 10 µg/ml pH 7.2. Protein concentrations were determined using the Bradford protein assay. Thirty micrograms of protein were resuspended in sodium dodecyl sulfate (SDS)-bromophenol blue loading buffer with 0,5 M dithiothreitol. The samples were separated on 8% SDS-polyacrylamide gels (Amersham Bioscience, Inc., Little Chalfont, England) and after electrophoresis (Mini-PROTEAN 3 System, Bio-Rad, Hercules, CA, USA), the proteins were transferred to nitrocellulose membranes (Amersham Bioscience) using a system of mini transblot cell (BioRad) overnight. After transfer, blots were incubated in a solution (blocking solution) containing Tris-buffered saline (TBS), 10% (w/v) Tween-20, 1% (w/v) non-fat milk and 1% (w/v) bovine serum albumin. Subsequently, blots were incubated overnight with rabbit anti-mGluR1a (1:1000), anti-mGluR5 (1:1000), anti-mGluR2/3 (1:1000), anti-NR1 (1:1000) and mouse antiGlu1 and antiGlu2 (1:500) (Upstate Biotechnology, Lake Placid, NY, USA) in blocking solution at 4°C. After incubation with the primary antibody, the blots were incubated with horseradish peroxidase-conjugated goat anti-rabbit or anti-mouse antibodies (1:5000; Amersham Bioscience) for 1h at room temperature (21°C ± 2). To ensure that each lane was loaded with an equivalent amount of protein, the blots were probed with an anti-actin serum (1:1000; Sigma, St Louis, MO, USA) overnight at 4°C. Subsequently, blots were incubated with horseradish peroxidase-conjugated goat anti-mouse antibodies (1:5000; Amersham Bioscience) for 1h at room temperature. Immunoreactive bands were visualized with an enhanced chemiluminescence system (Amersham Biosciences). After immunoblotting, digitized images of bands immunoreactive for target (mGluR1, mGluR5, mGluR2/3, NR1, Glu1 or Glu2) and control (actin) molecules were acquired and the area of immunoreactivity corresponding to each band was measured using the NIH Image medical imaging software. A ratio of target to actin was then determined, and these values were compared for statistical significance.

### Water Maze procedure

Mice at PND60 were tested in the Morris water maze for their spatial learning ability. The apparatus consisted of a circular pool (diameter 110 cm, height 60 cm) located in a test room with white walls with several cues on them. The pool, with its inner surface painted black, was filled to a depth of 40 cm with water (maintained at 25 ± 1°C), covering an invisible (black) 10-cm square platform. The platform was located approximately 0.5 cm below the surface of the water. The pool was virtually divided into 4 quadrants (North, South, East, or West) and the platform placed at a fixed position in the center of the North quadrant. Subjects received, in a single-day training procedure, four four-trial sessions of training, with each session separated by 30 min. Trials within each session were separated by 60 sec. On each trial, the subject was gently released in the water with its head facing the pool wall from one of quadrants. The order of the starting quadrant was changed in each session and trial. A maximum of 60 sec was allowed, during which the mouse had to find the platform, climb onto it and allowed to remain there for 10 sec. If the animal did not find the platform, it was gently guided with a grid and allowed to stay for 10 sec.

A video camera above the center of the pool was connected to a computerized tracking system that recorded and analyzed animal behavior (San Diego Instruments). The time of escape onto the platform and the swim speed were measured. After the last trial of the last session of training, animals were submitted to a single 60 sec "probe trial" in which the platform was removed from the pool. The animal started the probe trial in the south quadrant and the time that it swam through the north quadrant, where the platform had been previously located, provided a measure of learning accuracy in recalling the former position of the platform. A session with a visible platform was performed to assess the swimming speed of the two groups of animals.

### Corticosterone secretion after a restraint stress

On PND60 in the morning (9:00 AM) we examined corticosterone secretion before (basal value) and after 15 min of restrain stress. Blood samples were collected from the tail tip in heparinized capillary tubes. Restraint stress was carried out in a Plexiglass restraint device. Blood samples were centrifuged at 1900 x g at 4°C for 20 min; plasma was removed and kept frozen at -20°C until assay.

### Corticosterone assay

Plasma corticosterone concentrations were determined by radioimmunoassay (MP Biomedicals, France). The cross-reactivity of the polyclonal corticosterone-antisera with respective related substances was negligible. The inter- and intra-assay coefficients of variance were 7% and 4%, respectively, with a detection limit of 0.01µg/100ml.

### F2-isoprostane measurement

On PND0 the levels of the *F2-isoprostane 15-F₂ₜ-IsoP (also known as 8-epi-PGF*_{*2*α}*)* were measured in brain homogenates, as previously described (Minghetti et al., 2000). Briefly, brains were weighed and homogenized in 50 mM Tris buffer, pH 7.5 (1 mg/0.1 ml), containing the anti-oxidant 10µM BHT to block spontaneous oxidation. Homogenates were vigorously vortexed and incubated for 5 min on ice before centrifuging at 14,000 rpm for 45 min at 4 °C. Supernatants were collected and stored at -80 °C until required. 15-F₂ₜ-IsoP (also known as 8-epi-PGF2α) was measured by a specific enzyme immunoassay (Cayman Chemical, Ann Arbor, MI, USA), according to the manufacturer's instructions. Detection limit was 2 pg/ml; anti-15-F2t-IsoP antibody cross-reactivity with other iso-prostaglandins was less than 0.15%.

### Detection of Oxidized Proteins

On PND60 protein oxidation were measured in the same homogenates utilized for western blot of glutamate receptors. Oxidized protein was detected by using an oxidized protein detection kit (OxyBlot, Chemicon International). The OxyBlot provides reagents for sensitive immunodetection of carbonyl groups, which is a hallmark of the oxidation status of proteins. The procedure was performed according to manufacturer's recommendation. Briefly, 30 µg of hippocampal homogenates were derivatized with or without 2,4-dinitrophenylhydrazine (DNPH) and samples loaded onto a 12% SDS-PAGE gel. After separation, proteins were electrotransferred to a nitrocellulose membrane and incubated with a primary antibody against the derivatized carbonyl groups followed by incubation with a horseradish peroxidase-conjugate goat anti-rabbit antibody. The oxidized proteins were visualized by using an enhanced chemiluminescence system (Amersham Biosciences). The relative optical densities were quantified using the NIH Image medical imaging software. No immunoreactivity was detected in the non-DNPH derivatized brain homogenates

### Statistical analysis

Body weight data were analyzed separately for each age point by one-way ANOVA. Latencies to reach the platform in the Morris water maze were analyzed by three-way ANOVA for repeated measures (treatment x trial x session with repeated measures on trials and sessions). The ANOVA analyses were always followed by Fisher's LSD *post-hoc* comparisons. Probe trial, swimming speed, plasma corticosterone concentrations and immunoblot data were analyzed by Student's t-test. The level of significance was set at p<0.05.

### Effect of AZT and ALC treatments on body weight

In Table 1 the body weight of the litter mates at different age of life is described. As shown, the AZT prenatal treatment induces, at birth (PND0), a significant decrease in body weight (main treatment effect F_{3.36} =11.58, p<0.05; AZT vs Control, p<0.05 Fisher's LSD *post-hoc),* an effect that was not prevented by concomitant administration of ALC in the AZT+ALC group (AZT+ALC vs Control, p<0.05 Fisher's LSD *post-hoc).* ALC per se did not modify pups' body weight at birth, but increased it on PND24 (ALC vs all other groups, p<0.05 Fisher's LSD *post-hoc).* At PND60, mean body weight was comparable in the four experimental groups.

**Table 1**

| **Effect of AZT and ALC treatments on body weight at different ages in male litter mates** | | | | |
|---|---|---|---|---|
| | Control | AZT | ALC | AZT + ALC |
| PND0 | 1.83 ± 0.04 | 1.63 ± 0.03 * | 1.89 ± 0.06 | 1.57 ± 0.05* |
| PND24 | 15.01 ± 0.81 | 14.49 ± 0.68 | 17.04 ± 0.77# | 14.43 ± 0.51 |
| PND60 | 38.69 ± 1.17 | 37.04 ± 1.75 | 39.71 ± 1.13 | 37.46 ± 1.24 |

| | | | | |
|---|---|---|---|---|
| p<0.05 vs Control); # p<0,05 vs all other groups). Values are expressed as mean ± S.E.M (n= 9-11 mice per group) | | | | |

### Effect of AZT and ALC treatments on Expression of hippocampal glutamate receptors in two months old mouse (PND60).

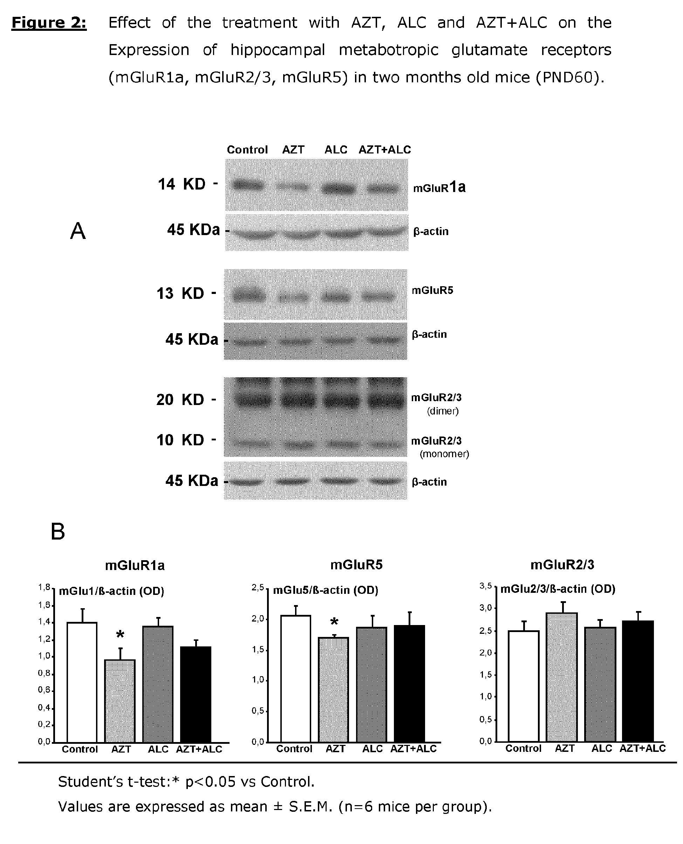

The AZT prenatal treatment caused in adult mice a significant reduction of mGluR1a receptor expression with respect to control mice (p<0.05, Student's t-test, t= -2.32) (Fig. 2). Interestingly, the mGluR1a receptors in AZT+ALC group showed a trend to increase (although not statistical significant) in respect to AZT animals and did not differ from control group. Therefore, ALC treatment could be able to prevent the AZT-induced reduction of mGluR1a receptor expression. This phenomenon, although less evident, was also observed for mGluR5 receptor expression: the AZT group showed a reduction of receptor expression compared to control group (p<0.05, Student's t-test, t= -3.17), while the AZT+ALC group showed a trend to increase.

With regard to the mGluR2/3 receptor expression, no differences between groups were found.

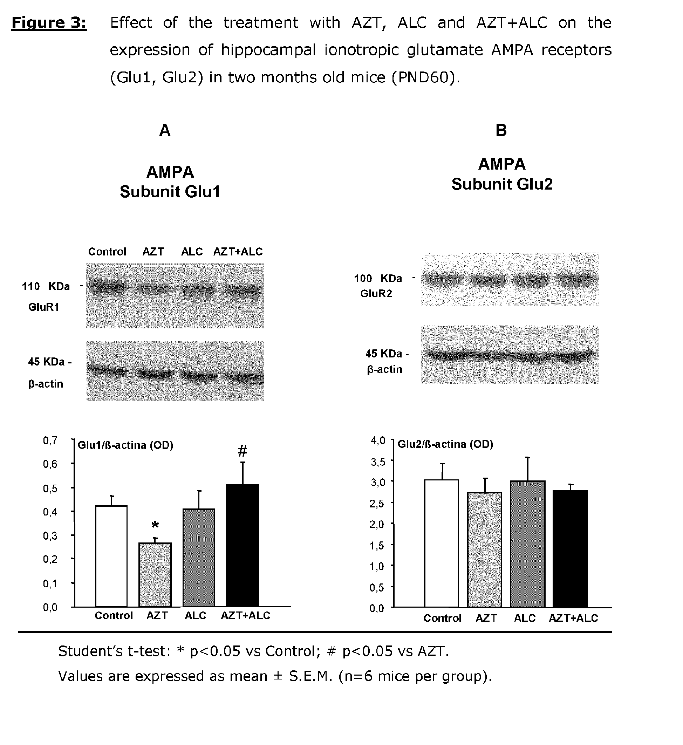

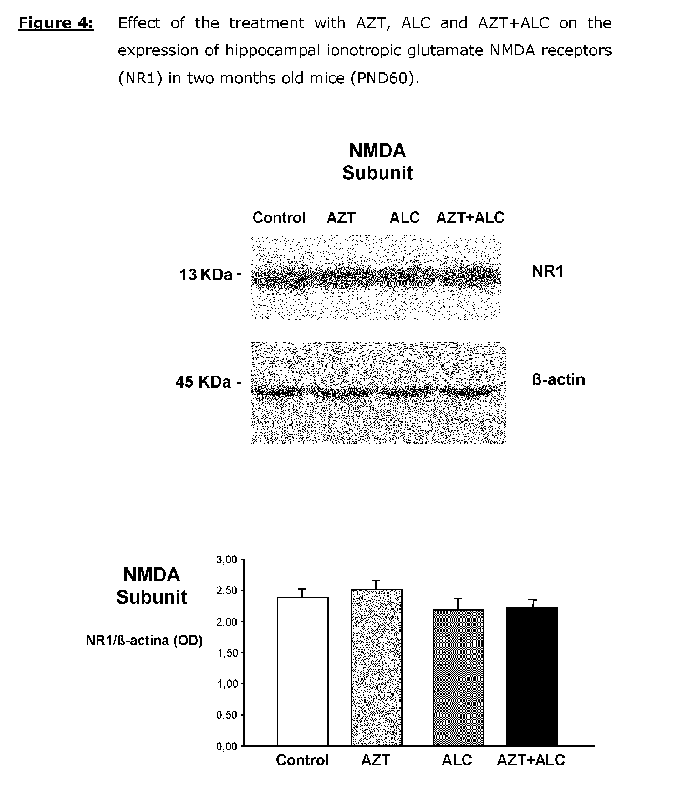

In Fig. 3 protein expression relative to ionotropic receptors is shown. The analysis of Student's t-test has shown that AZT prenatal treatment caused a significant reduction of Glu1 subunit of AMPA receptor expression in respect to control mice (p<0.05, Student's t-test, t= -3.43) (Figure 3A). Interestingly, the Glu1 subunit in AZT+ALC group showed a significant increase (p<0.05, Student's t-test, t= -2.59) with respect to AZT mice and did not differ from Controls suggesting that again the ALC treatment could prevent the reduction of receptor expression due to the AZT treatment. With regard to the AMPA Glu2 subunit (Figure 3B) and NMDA NR1 subunit (Figure 4), no differences between groups were found.

### Effect of AZT and ALC treatments on spatial learning test in two months old mouse (PND60).

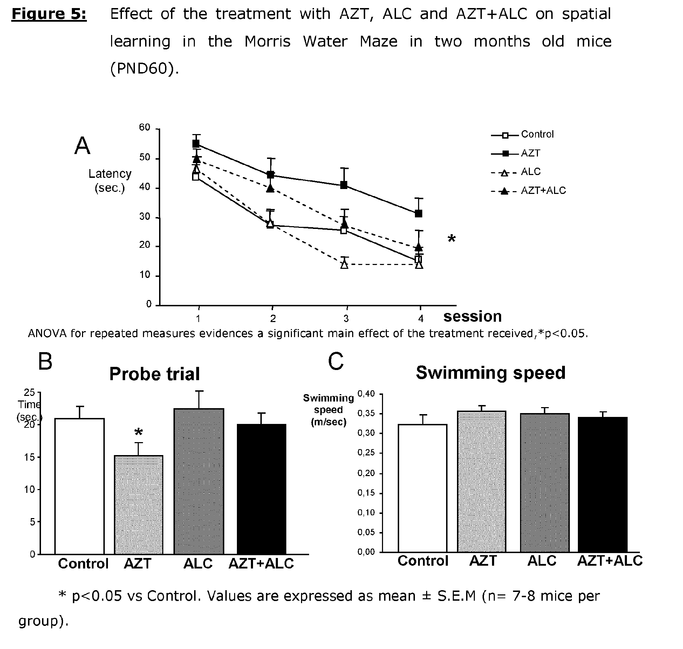

Figure 5A shows the latencies to reach the hidden platform throughout the four sessions of the MWM. ANOVA for repeated measures evidenced a significant main effect of the treatment received (F_{3.26} = 5.95, p<0.05) and a significant three way interaction treatment x session x trial (F_{3.27} = 1.89, p<0.05). Post-hoc analysis showed that subjects that have received prenatal AZT treatment display a significant increase in escape latency in respect to the control group in all the four sessions. The AZT+ALC group has, in the 3^{rd} and 4^{th} session, escape latency comparable to the Control group, and significantly different from the AZT group, suggesting that pre-treatment with ALC improved the AZT-induced cognitive deficits. Finally, ALC treatment alone did not modify learning abilities.

During the probe trial (Fig. 5B), AZT subjects spent less time than control mice in the quadrant in which the hidden platform was present before being removed from the maze (t= -2.05, p<0.05), whereas AZT+ALC mice did not differ from controls. No treatment-induced difference was recorded in the swimming speed as demonstrated by the similarity between groups (Fig. 5C).

### Effect of AZT and ALC treatments on stress corticosterone secretion in two months old mouse (PND60).

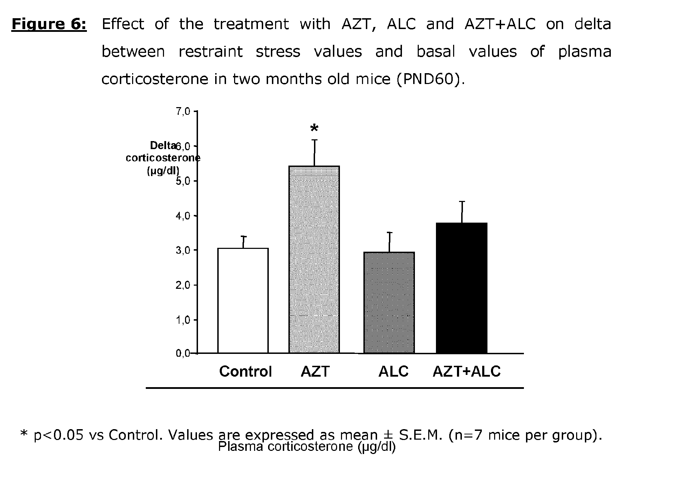

In basal conditions, no statistical differences were observed in plasma corticosterone levels between groups, while the one-way Anova analysis of plasma corticosterone stress response, calculated as delta between restraint stress values and basal values, reveals a significant difference between groups (p<0.05). The Fisher's test highlights that the AZT group has a higher plasma corticosterone concentration in respect to all other animal groups. What is remarkable is that the AZT+ALC group show levels of corticosterone similar to the control group indicating that the prenatal treatment with ALC had an important effect on the development of HPA axis (Figure 6).

### Effect of AZT and ALC treatments on oxidative stress markers in newborn (PND0) and two months old mice (PND60).

Occurrence of free radical generation and oxidative stress was monitored at birth and at 2 months of age.

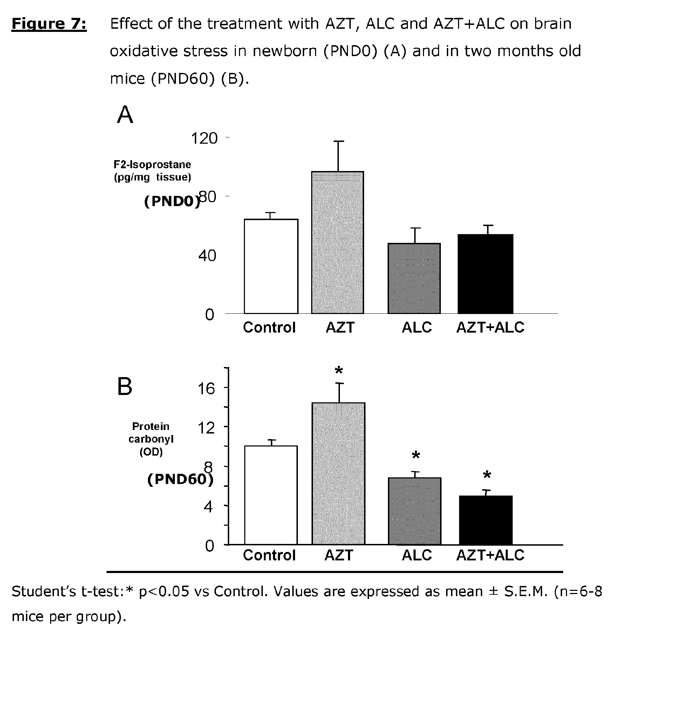

At PND0, the levels of 15-F2t-Isop, a reliable and sensitive marker of oxidative stress, were tested in whole brain homogenates (Fig. 7A). Consistent with the oxidative stress hypothesis of AZT toxicity, 15-F2t-Isop levels showed a trend to increase in homogenates from AZT prenatally-treated pups, but not in those from ALC or ALC+AZT groups.

At PND60, protein carbonyl levels, which are considered a measure of protein oxidation and an index of oxidative stress, were measured by OxyBlot analysis of homogenates prepared from hippocampi of AZT and ALC-treated mice. In agreement with the tendency observed at PND0, a significant increase in carbonyls was found in the hippocampal homogenates from AZT prenatally-treated mice (p<0.05, Student's t-test, t= 2.15; Fig. 7B). Significant decreases of protein carbonyls were detected in the hippocampus of mice treated with ALC and with the combination of AZT + ALC, as expected due to the antioxidant effect of ALC (p<0.05, Student's t-test, t= -3.45 for ALC, t= -5.07 for AZT + ALC; Fig. 7).

## Claims

1. Acetyl L-carnitine or one of its pharmaceutically acceptable salts for use for preventing cognitive deficit disorders in a new born from HIV-seropositive pregnant female who is in treatment with azidothymidine.

2. Acetyl L-carnitine or one of its pharmaceutically acceptable salts according to claim 1, in which the pharmaceutically acceptable salt of acetyl L-carnitine is selected from the group consisting of chloride, bromide, orotate, aspartate, acid aspartate, citrate, acid citrate, magnesium citrate, phosphate, acid phosphate, fumarate, acid fumarate, magnesium fumarate, glycerophosphate, lactate, maleate and acid maleate, mucate, oxalate, acid oxalate, pamoate, acid pamoate, sulphate, acid sulphate, glucose phosphate, tartrate, acid tartrate, magnesium tartrate, 2-amino ethanesulphonate, magnesium 2-amino ethanesulphonate, methanesulphonate, choline tartrate, trichloroacetate, and trifluoroacetate.

3. Acetyl L-carnitine or one of its pharmaceutically acceptable salts according to claim 1, in which acetyl L-carnitine is administered in concomitant or sequential manner respect to AZT.

4. Acetyl L-carnitine or one of its pharmaceutically acceptable salts according to claim 1, in which acetyl L-carnitine is administered enterally or parenterally such as subcutaneous, intravenous, intradermal, intramuscular, intraperitoneal, intranasal, transdermal, oral, by bolus injection or perfusion route.

5. Acetyl L-carnitine or one of its pharmaceutically acceptable salts according to claim 1, in which acetyl L-carnitine is administered in the form of tablets, capsules, suppositories, suspensions or solutions.

6. Acetyl L-carnitine or one of its pharmaceutically acceptable salts according to claim 1, in which acetyl L-carnitine is administered at a dose of 0.5-4 g/day, a preferred dose being 1-3 g/day, the most preferred dose being 2 g/day.
